# EUROPEAN PATENT SPECIFICATION

(11) **EP 2 441 376 B1**
(45) Date of publication and mention of the grant of the patent: **04.07.2018**
(21) Application number: 11184715.8
(22) Date of filing: 11.10.2011
(51) Int. Cl.: A61B 1/00, A61B 8/00

(54) **SWITCHING VALVE ASSEMBLY FOR ENDOSCOPE**
SCHALTVENTILANORDNUNG FÜR ENDOSKOP
ENSEMBLE FORMANT SOUPAPE DE COMMUTATION POUR ENDOSCOPE

(30) Priority: 12.10.2010 JP 2010229508
(43) Date of publication of application: 18.04.2012
(73) Proprietor: Fujifilm Corporation, Minato-ku Tokyo (JP)
(72) Inventor: MORIMOTO, Yasuhiko, Kanagawa (JP)
(74) Representative: Klunker IP Patentanwälte PartG mbB

(56) References cited:
- DE-A1-102004 003 857
- JP-A- 7 008 449
- JP-A- 8 238 212
- JP-A- 2003 305 003
- JP-A- 2006 175 175
- JP-A- 2009 045 126
- US-A- 4 261 343
- US-A- 4 765 312
- US-A- 4 800 869
- US-A- 5 840 016
- US-A- 5 871 441
- US-A1- 2004 238 014
- US-A1- 2010 049 001

## Description

### BACKGROUND OF THE INVENTION

### 1. Field of the Invention

The present invention relates to a switching valve assembly for an endoscope according to the preamble of claim 1. More particularly, the present invention relates to a switching valve assembly for endoscope, in which a piston unit can be rotationally regulated inside a cylinder by use of a compact structure and without influence to performance in flow.

### 2. Description Related to the Prior Art

Ultrasonic examination is known in the medical field. Ultrasonic waves are applied to a body, from which reflected waves or echo is received to form an image of an object in the body. An example of the ultrasonic examination is an endoscopic examination, in which an instrument with ultrasonic transducers is entered in a body cavity and emits the ultrasonic waves. The endoscopic examination is advantageous in comparison with examination with the ultrasonic waves percutaneously, because a condition of tissue of a stomach, large intestine or other organs can be imaged very precisely. Importance of the endoscopic examination is typically great for precise diagnosis of a tumor, ulcer and the like of a body cavity, in relation to their depth and other attributes.

For the endoscopic examination, an ultrasonic endoscope or an ultrasonic probe is used. The ultrasonic endoscope has an array of ultrasonic transducers, CCD as an image sensor and the like incorporated in a head assembly. The ultrasonic probe is used by entry in an instrument channel of an endoscope. In the field of ultrasonic imaging, there is a serious problem of attenuation of the ultrasonic waves in the presence of air for application of the ultrasonic waves from the tip of the ultrasonic endoscope or the ultrasonic probe to a wall of a body cavity. In view of this problem, a balloon is disposed at the tip of the ultrasonic endoscope or the ultrasonic probe for covering the ultrasonic transducers. The balloon is charged with water as a transmission medium and expanded, and then set in tight contact with the wall of the body cavity. Then the ultrasonic waves are emitted from the inside of the balloon. Thus, attenuation of the ultrasonic waves with air is prevented. When the endoscopic examination is completed, water is drained from the balloon to compress the balloon, before the ultrasonic endoscope is removed easily from the body.

An air/water supply button assembly and a suction button assembly are disposed on a handle device of the ultrasonic endoscope. Expansion and compression of the balloon are changed over by pushing the air/water supply button assembly and the suction button assembly. In general, a two-step button is used as an example of each of the air/water supply button assembly and the suction button assembly.

JP-A 10-028670 (corresponding to JP-B 3017957) and JP-A 2007-111266 disclose the air/water supply button assembly, which includes a button device or top cap device, and a vent formed in the button device. When an air supply pump is driven without pushing the air/water supply button assembly, air is leaked through the vent in the button device. When the vent is closed, a check valve is opened in the air/water supply button assembly, so that an air/water supply nozzle ejects air, because the vent is closed in the air supply state. When the button device is depressed halfway, the operation is changed over from the air supply to the water supply. The air/water supply nozzle ejects water. When the button device is depressed fully, the water channel is changed over. A balloon channel supplied water to the balloon for expansion.

A suction pump operates for suction of air. In an inactive condition of the suction button assembly according to JP-A 10-028670 and JP-A 2007-111266, the suction pump draws external air through a vent channel. When the button device of the suction button assembly is depressed halfway, an instrument channel comes to communicate with the suction pump, and operates for the suction. When the button device is depressed fully, the suction pump comes to communicate with the balloon channel. Water is drained from the balloon through the balloon channel to compress the balloon.

The suction button assembly as two-step button includes a cylinder, a piston, the button device, and a housing wall. The piston is contained in the cylinder in a slidable manner. There is an end opening of a cylinder passage formed in the cylinder, at which an upper end of the piston protrudes. The button device is formed with the upper end of the piston. The housing wall is disposed to cover the end opening, and keeps the button device operable for depression to a halfway position and a down position.

Plural channels are connected to the cylinder passage, including a discharge conduit, a suction channel and a balloon compression channel. The discharge conduit extends to the suction pump. The suction channel extends to the instrument channel. The balloon compression channel extends to the balloon channel. A valve head or piston rod or lower stem end is a lower end of the piston. A flow channel is formed through the valve head to extend between a side wall and a lower end point of the valve head. Also, a groove is formed in the side wall of the valve head. When the button device is depressed halfway, the flow channel and the groove operate for the discharge conduit to communicate with the suction channel. When the button device is depressed fully, the flow channel and the groove operate for the discharge conduit to communicate with the balloon compression channel. To this end, the flow channel and the groove are conditioned for their position and shape.

In the suction button assembly described above, a flow channel width between one flow opening and a valve opening of a flow channel of the valve head for fluid may be very small if the valve opening of the flow channel is not aligned with the flow opening of a discharge conduit or a suction channel formed in an inner wall of a cylinder passage upon a halfway depression of a button device. Fluid containing particles of a relatively large size cannot be passed with this flow channel width. In view of this problem, an anti-rotation projection is formed on the side wall of a piston unit. An engagement groove is formed in the inner wall of the cylinder passage, extends in the longitudinal direction of the piston unit, and is engaged with the anti-rotation projection in a slidable manner. Thus, it is possible to prevent the piston unit from rotating relative to the cylinder by keeping one of the flow openings of the cylinder passage aligned with the valve opening of the flow channel when the button device is depressed halfway. Performance of suction of the suction button assembly can be maximized. For example, a size of particles of fluid passable in the suction button assembly can be enlarged.

In the suction button assembly of the two-step structure, the discharge conduit is set in series only with the suction channel upon halfway depression of the button device, and is set in series only with the balloon compression channel upon full depression of the button device. It is necessary to form a plurality of the grooves or the flow channel in a side wall of the piston unit. It is likely that no sufficient space can be reserved for forming the anti-rotation projection according to a small length of the piston unit. Furthermore, there is a problem in excessive complexity of the grooves of the flow channel or an excessively large size of the piston unit due to disposing the anti-rotation projection on the side wall of the piston unit. Performance of the suction of the suction button assembly will be seriously lower due to reduction in the diameter of the piston unit or the cylinder passage if the anti-rotation projection is formed on the piston unit or the grooves are formed in the inner wall of the cylinder passage.

In accordance with the preamble of claim 1, US 4 261 343 A discloses a switching wave assembly in which an axial groove is provided within a lower part of the piston unit and a locking screw is inserted through a hole within the cylinder wall, the tip end of the screw being engaged with said groove in order to prevent a relative rotation between the piston and the cylinder.

### SUMMARY OF THE INVENTION

In view of the foregoing problems, an object of the present invention is to provide a switching valve assembly for endoscope, in which a piston unit can be rotationally regulated inside a cylinder by use of a compact structure and without influence to performance in flow.

In order to achieve the above and other objects and advantages of this invention, a switching valve assembly for an endoscope includes a cylinder having a cylinder passage and a plurality of flow openings formed inside the cylinder passage, plural flow lines extending from respectively the flow openings. In the switching valve assembly, a piston unit is contained in the cylinder passage in a slidable manner, the piston unit including a valve head, a flow channel formed in the valve head, an end rod, formed to extend from the valve head in an upward direction, for protruding from the cylinder passage, and a button device formed on a tip of the end rod. The piston unit slides to an initial position, a down position lower than the initial position and where the button device is positioned nearest to the cylinder passage, and a halfway position defined between the initial position and the down position, for changing a combination of the flow openings with the flow channel to change over communication and interruption between the flow lines . A biasing means of a two-stage type is secured to the cylinder on an upper side of the cylinder passage, for biasing the piston unit in the upward direction from the down position, the biasing means having smaller force of bias above the halfway position than under the halfway position, for stopping the piston unit in the halfway position when the button device is pushed from the initial position and slid in a downward direction. A first regulating means prevents the piston unit from rotating relative to the biasing means. A second regulating means prevents the biasing means from rotating relative to the cylinder, so as to ensure alignment of a channel opening of the flow channel with one of the flow openings upon sliding the piston unit.

The biasing means includes a cylinder cap device, secured to the cylinder under the button device, for partially covering the piston unit. A slide device of a cup shape is contained in the cylinder cap device, having a receiving hole, for receiving entry of the piston unit, for guiding the piston unit in a longitudinal direction thereof, and for sliding in the downward direction in the cylinder cap device when the button device is pushed in the downward direction. Air-tight packing is fitted on the slide device, for contacting an inner surface of the cylinder cap device in an air-tight manner. A first biasing mechanism is disposed between the slide device and the cylinder cap device, for biasing the slide device in the upward direction from the cylinder . A second biasing mechanism is disposed between the button device and the slide device, for biasing the button device in the upward direction with smaller force of bias than the first biasing mechanism.

The first and second biasing mechanisms are compression coil springs.

Furthermore, a third regulating means prevents the slide device from rotating relative to the cylinder cap device. The first regulating means prevents the piston unit from rotating relative to the slide device, and the second regulating means prevents the cylinder cap device from rotating relative to the cylinder.

The first regulating means includes a first regulating flat surface formed by chamfering a peripheral surface of the end rod. A second regulating flat surface is formed inside the receiving hole, for tightly contacting the first regulating flat surface for engagement.

The third regulating means includes a first regulating projection, formed to project from a bottom plate of the cylinder cap device in the upward direction. A first regulating recess is formed in an inner wall of the slide device, and engaged with the first regulating projection.

The second regulating means includes a second regulating projection, formed to project from the bottom plate of the cylinder cap device in the downward direction. A second regulating recess is formed in the cylinder, and engaged with the second regulating projection.

The first regulating projection is disposed collinearly with the second regulating projection in the longitudinal direction of the piston unit.

Furthermore, an end ring is secured inside the cylinder cap device, for keeping the slide device contained in the cylinder cap device.

Furthermore, a guide sleeve is disposed on an upper wall of the slide device, having the receiving hole, for extending long in the longitudinal direction of the piston unit.

The valve head has a larger diameter than a diameter of the receiving hole.

The button device includes a button head secured to the tip of the end rod. A cap top portion covers an upper surface of the button head.

The down position of the piston unit is adapted to expansion or compression of a balloon of the endoscope with water, and the halfway position of the piston unit is adapted to supply or suction of fluid through a nozzle of the endoscope in a body cavity.

Consequently, a piston unit can be rotationally regulated inside a cylinder by use of a compact structure and without influence to performance in flow, because the first and second regulating devices cooperate for the rotational regulation of the piston unit.

### BRIEF DESCRIPTION OF THE DRAWINGS

The above objects and advantages of the present invention will become more apparent from the following detailed description when read in connection with the accompanying drawings, in which:
Fig. 1 is an explanatory view in a section, illustrating an endoscope;
Fig. 2 is a vertical section illustrating a suction button assembly;
Fig. 3 is a front elevation illustrating the suction button assembly;
Fig. 4 is a top plan illustrating a cylinder;
Fig. 5 is a perspective view illustrating a cylinder cap device;
Fig. 6 is a bottom perspective view illustrating the cylinder cap device;
Fig. 7 is a perspective view illustrating a slide device of a cup shape;
Fig. 8 is a bottom perspective view illustrating the slide device;
Fig. 9 is a cross section illustrating the suction button assembly taken on line IX-IX in Fig. 2;
Fig. 10 is a cross section illustrating the suction button assembly taken on line X-X in Fig. 2;
Fig. 11 is a cross section illustrating the suction button assembly taken on line XI-XI in Fig. 2;
Fig. 12 is a chart illustrating relationships between the cylinder and the piston unit for rotational regulation;
Fig. 13 is a vertical section illustrating the suction button assembly in an initial state of the button device;
Fig. 14 is a vertical section illustrating the suction button assembly in a state of halfway depressing the button device;
Fig. 15 is a vertical section illustrating the suction button assembly in a state of fully depressing the button device.

### DETAILED DESCRIPTION OF THE PREFERRED EMBODIMENT(S) OF THE PRESENT INVENTION

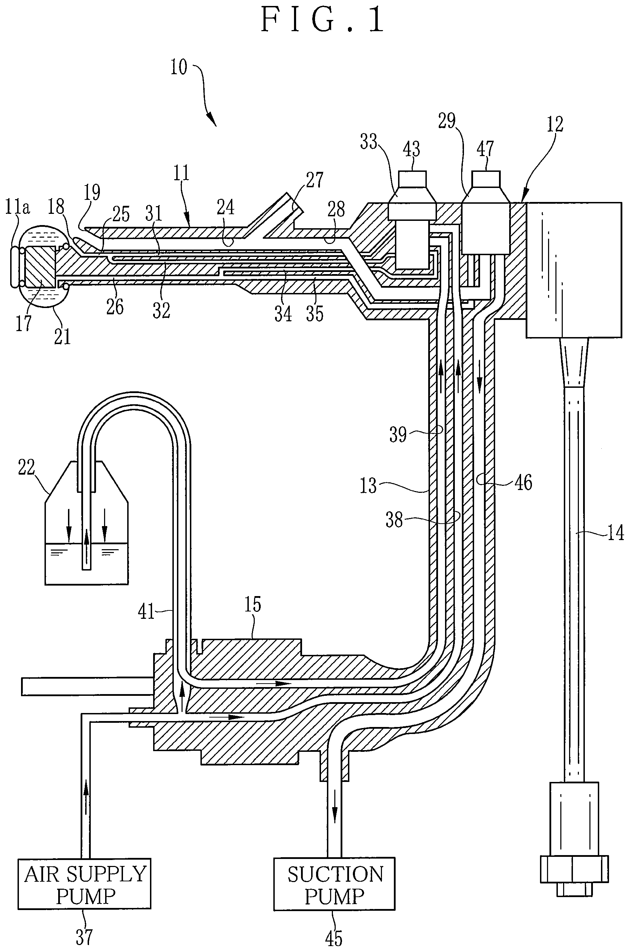

In Fig. 1, an ultrasonic endoscope 10 includes a section of an elongated tube 11, a handle device 12, a universal cable 13 or connection tube, and a connection cable 14. The elongated tube 11 is entered in a body cavity of a patient. The handle device 12 is a basic portion at which the universal cable 13 and the connection cable 14 are connected to the handle device 12. A connector 15 is disposed at a proximal end of the universal cable 13 for connection with a processing apparatus for the endoscope with a light source apparatus. A proximal end of the connection cable 14 is connected to the processing apparatus (not shown).

The elongated tube 11 has a circular shape as viewed in a cross section, and is flexible. There is a head assembly 11a of the elongated tube 11. The head assembly 11a includes an ultrasonic transducer array 17, a CCD image sensor (not shown), an air/water supply nozzle 18, and a suction nozzle 19 or instrument opening. The ultrasonic transducer array 17 operates to form an ultrasonic image. The CCD image sensor forms an endoscopic image. The air/water supply nozzle 18 washes an objective system for imaging (not shown). The suction nozzle 19 is a distal opening for protrusion of a medical instrument such as a forceps, and also is a suction opening for drawing fluid such as blood or body fluid.

A resilient balloon 21 is secured to the head assembly 11a in a removable manner. The balloon 21 is initially compressed to contact the outer surface of the head assembly 11a tightly before entry into the body cavity. A water supply source 22 or water supply tank supplies water to the balloon 21 for expansion for the purpose of applying ultrasonic waves from the ultrasonic transducer array 17. The balloon 21 operates for tightening the contact of the head assembly 11a on a wall of the body cavity, and prevents ultrasonic waves and echo from attenuating with air. After the expansion, the balloon 21 is compressed again by discharge of water. An example of material of the balloon 21 is latex rubber and the like.

An instrument channel 24, a fluid supply channel 25 and a balloon channel 26 are formed through the elongated tube 11 and the handle device 12. A distal end of the instrument channel 24 is the suction nozzle 19. A distal end of the fluid supply channel 25 is the air/water supply nozzle 18. A distal end of the balloon channel 26 extends to the inner space of the balloon 21. Note that in Fig. 1, portions other than the channels are hatched for the purpose of clarifying the channels.

A proximal instrument opening 27 is formed in the elongated tube 11, and is an open end of the instrument channel 24. A seal cap (not shown) is fitted on the proximal instrument opening 27 for closing except for entry of a medical instrument or forceps. A suction channel 28 is a branch conduit of the instrument channel 24. A suction button assembly 29 or switching valve assembly is associated with the handle device 12. The suction channel 28 is connected to the suction button assembly 29.

An air channel 31 and a water channel 32 are branches extending from a proximal end of the fluid supply channel 25. On the handle device 12 is an air/water supply button assembly 33 or switching valve assembly, to which the air channel 31 and the water channel 32 are connected. A balloon expansion channel 34 and a balloon compression channel 35 are branches extending from a proximal end of the balloon channel 26. The balloon expansion channel 34 is connected to the air/water supply button assembly 33. The balloon compression channel 35 is connected to the suction button assembly 29.

There is an air supply conduit 38, which extends to an air supply source 37 or air supply pump. A water supply conduit 39 extends to the water supply source 22. Various flow lines are connected to the air/water supply button assembly 33, including the air channel 31, the water channel 32, the balloon expansion channel 34, the air supply conduit 38 and the water supply conduit 39. The air supply source 37 operates constantly during the ultrasonic imaging.

A branch conduit 41 is a branch from a proximal end of the air supply conduit 38 and extends through the connector 15. The branch conduit 41 is connected to a port of the water supply source 22. A proximal end of the water supply conduit 39 is disposed within the water supply source 22 by way of the branch conduit 41. Inner pressure of the water supply source 22 is raised by air supply through the branch conduit 41 from the air supply source 37. Water from the water supply source 22 is drawn to the water supply conduit 39.

The air/water supply button assembly 33 is a two-step button. A button device 43 or top cap device is an upper element of the air/water supply button assembly 33. A vent channel (not shown) is formed through the button device 43. When the button device 43 is not depressed, the air/water supply button assembly 33 shuts off the water supply conduit 39, and sets the air supply conduit 38 in series with the vent channel of the button device 43. Air from the air supply conduit 38 is leaked through the vent channel of the air/water supply button assembly 33. A finger of an operator closes the vent channel, to set the air supply conduit 38 in series with the air channel 31 in a state of shut-off of the water supply conduit 39, in a manner described in JP-A 10-028670 (corresponding to JP-B 3017957). Thus, the air is caused to flow to the air channel 31 and ejected through the air/water supply nozzle 18.

When the button device 43 is depressed halfway, the air/water supply button assembly 33 shuts off the air supply conduit 38, and sets the water supply conduit 39 in series with only the water channel 32. Water from the water supply conduit 39 is passed through the water channel 32 and ejected from the air/water supply nozzle 18. When the button device 43 is depressed fully, the air/water supply button assembly 33 sets the water supply conduit 39 in series with only the balloon expansion channel 34 while the air supply conduit 38 is kept shut off. Therefore, water from the water supply conduit 39 is passed through the balloon expansion channel 34 and supplied into the balloon 21.

There is a discharge conduit 46 having proximal and distal ends. The proximal end is connected to a suction pump 45. The distal end is connected to the suction button assembly 29 as well as the suction channel 28 and the balloon compression channel 35. The suction pump 45 operates for suction during the ultrasonic imaging. The suction button assembly 29 is a two-step button in a manner similar to the air/water supply button assembly 33.

When a button device 47 or top cap device is not depressed, the suction button assembly 29 sets the discharge conduit 46 open to the atmosphere. If the discharge conduit 46 is not open, load to the suction pump 45 may increase, as the suction pump 45 operates constantly. As the discharge conduit 46 is set open to the atmosphere, overload to the suction pump 45 can be prevented.

When the button device 47 is depressed halfway, the suction button assembly 29 sets the discharge conduit 46 in series only with the suction channel 28. Force of the suction with negative pressure rises in the suction channel 28 and the instrument channel 24 to draw fluid through the suction nozzle 19. When the button device 47 is depressed fully, the suction button assembly 29 sets the discharge conduit 46 in series only with the balloon compression channel 35. Thus, water is discharged from the balloon 21 by increasing the force of the suction with negative pressure in the balloon compression channel 35 and the balloon channel 26.

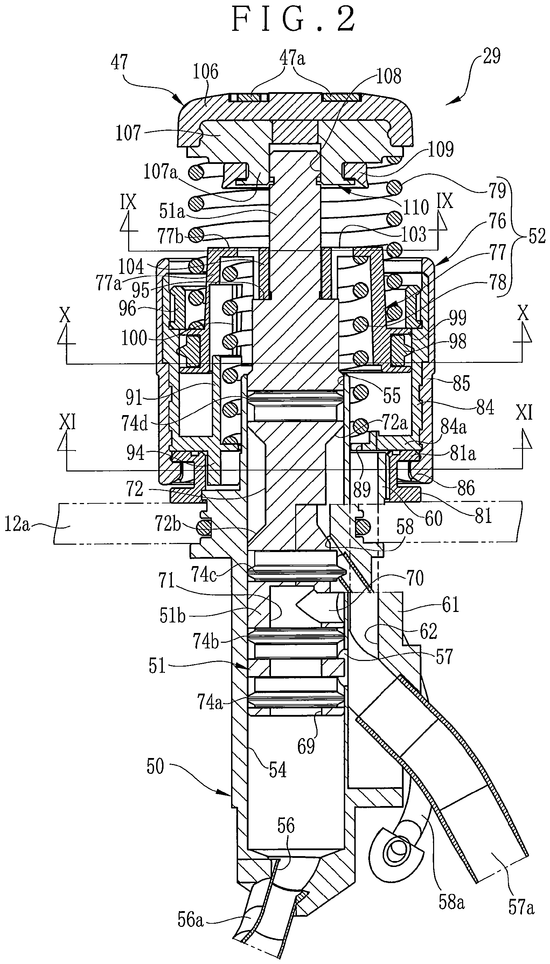

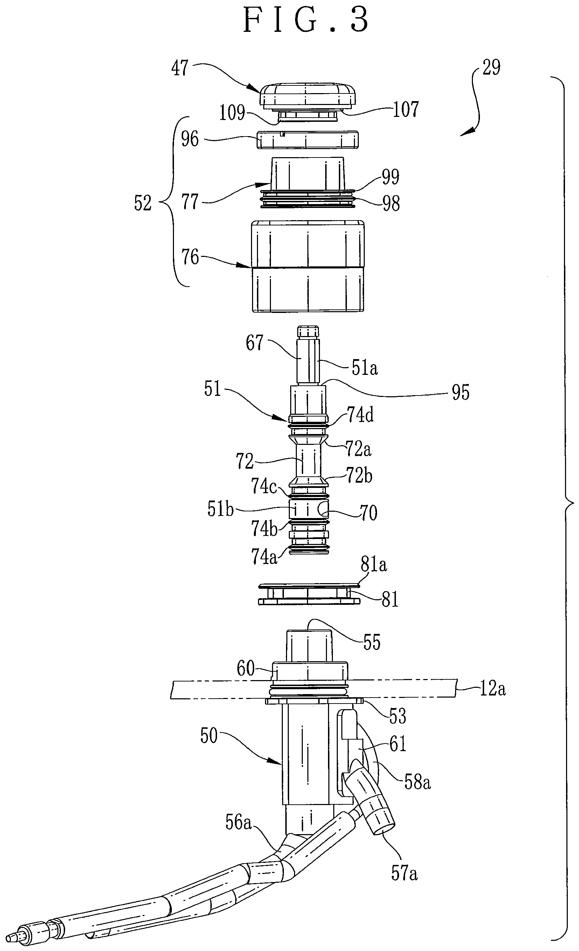

In Figs. 2 and 3, the suction button assembly 29 includes a cylinder 50, a piston unit 51, and a biasing unit 52 (as biasing means) of a cup shape or cylinder cap unit (housing unit). The cylinder 50 is firmly secured to the handle device 12. The piston unit 51 is contained in the cylinder 50 in a slidable manner. The biasing unit 52 is retained on the cylinder 50, and positions the piston unit 51 in a halfway position and a down position lower than the halfway position.

The cylinder 50 is formed from metal. The suction channel 28, the balloon compression channel 35, and the discharge conduit 46 are connected to the cylinder 50. A cylinder passage 54 of a multi-nozzle type is formed through the cylinder 50 and extends longitudinally. An end opening 55 is an open end of the cylinder passage 54. A flow port 56 or suction port is another open end of the cylinder passage 54, and communicates with the suction channel 28. A suction channel coupling 56a or nozzle is disposed to couple the flow port 56 to the suction channel 28.

A flow opening 57 or nozzle opening and a drain port 58 are formed in an inner wall of the cylinder passage 54. The flow opening 57 communicates with the discharge conduit 46 in a downward direction. The drain port 58 communicates with the balloon compression channel 35. A discharge channel coupling 57a or nozzle of the flow opening 57 is connected to the discharge conduit 46. A drain channel coupling 58a or nozzle of the drain port 58 is connected to the balloon compression channel 35.

A receiving threaded portion 60 is an end portion of the cylinder 50, and contacts a lower portion of the biasing unit 52. See Fig. 3. A discharge chamber 61 is disposed for connection of the flow opening 57 on the outside of the cylinder 50, and used for connection with the discharge channel coupling 57a. The discharge chamber 61 extends longitudinally toward an end of the cylinder 50, and is connected with the receiving threaded portion 60. In Fig. 2, the discharge chamber 61 is partially cut away for clarifying the connected portions of the cylinder 50 and the balloon compression channel 35.

A fluid channel 62 is formed through the discharge chamber 61. The flow opening 57 and the discharge channel coupling 57a are open at a lower end of the fluid channel 62. An upper end of the fluid channel 62 is connected to the receiving threaded portion 60 as indicated by the phantom line of Fig. 2.

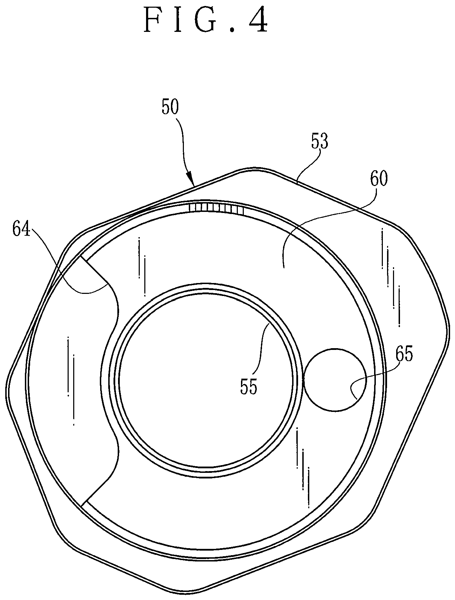

In Fig. 4, a regulating recess 64 is formed with the receiving threaded portion 60 by chamfering an outer wall, and operates for rotational regulation with the biasing unit 52. A cylinder vent hole 65 is formed in an upper wall of the receiving threaded portion 60, and communicates with the fluid channel 62. A male thread (not shown) is formed around the receiving threaded portion 60. A threaded ring 81 as a retaining ring is helically engaged with the male thread. See Fig. 3. A support flange 53 is formed with a lower portion of the receiving threaded portion 60. A handle housing 12a of the handle device 12 has a lower surface. The support flange 53 contacts the lower surface. The cylinder 50 is fixedly secured to the handle housing 12a by helical engagement of the threaded ring 81 with the male thread.

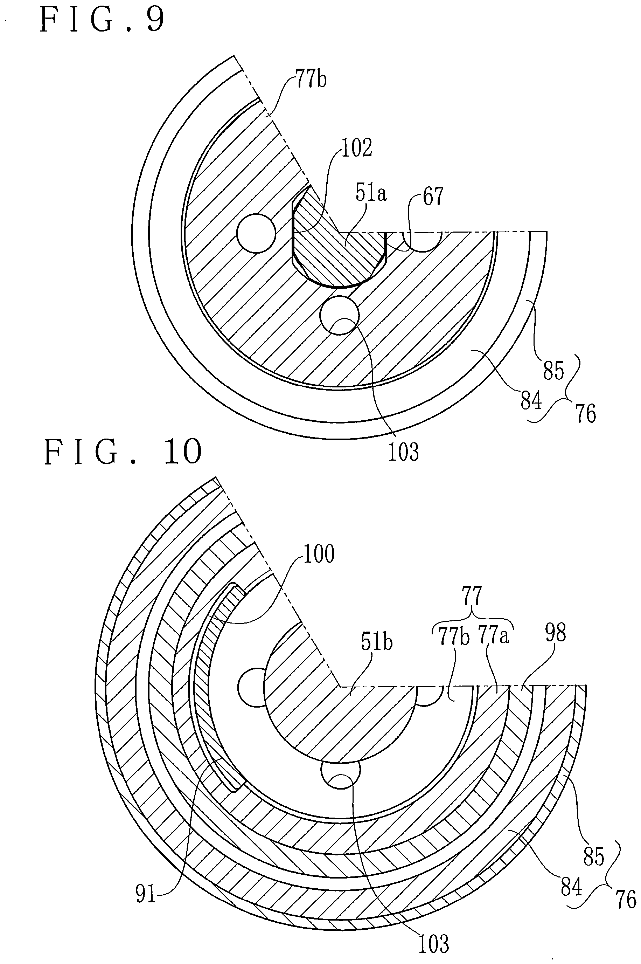

In Figs. 2 and 3, the piston unit 51 is formed from metal, and includes an end rod 51a, and a valve head 51b or piston rod or valve sleeve. The end rod 51a protrudes from the end opening 55. The valve head 51b is always contained in the cylinder passage 54. A diameter of the end rod 51a is smaller than that of the valve head 51b. A regulating flat surface 67 is formed by chamfering the end rod 51a, and operates for rotational regulation of the biasing unit 52. See Figs. 3 and 9.

The button device 47 is fixed on an upper end of the end rod 51a, has a disk shape, and is depressed for halfway depression and full depression. A target indicia 47a is formed on an upper surface of the button device 47 for expressing a depression position of a finger.

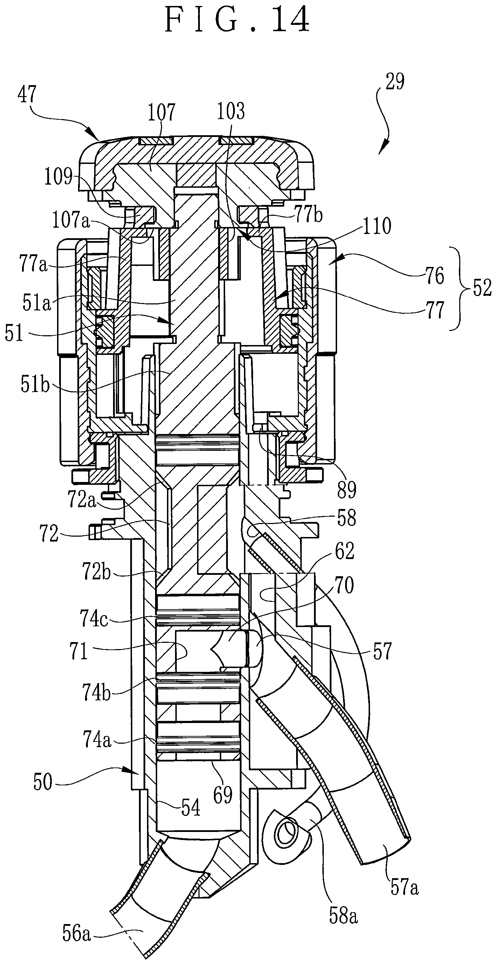

The valve head 51b includes a lower opening 69, a valve opening 70 or side opening, and a flow channel 71 for communication between the lower opening 69 and the valve opening 70. The valve opening 70 is so disposed as to be aligned with the flow opening 57 upon halfway depression. See Fig. 14.

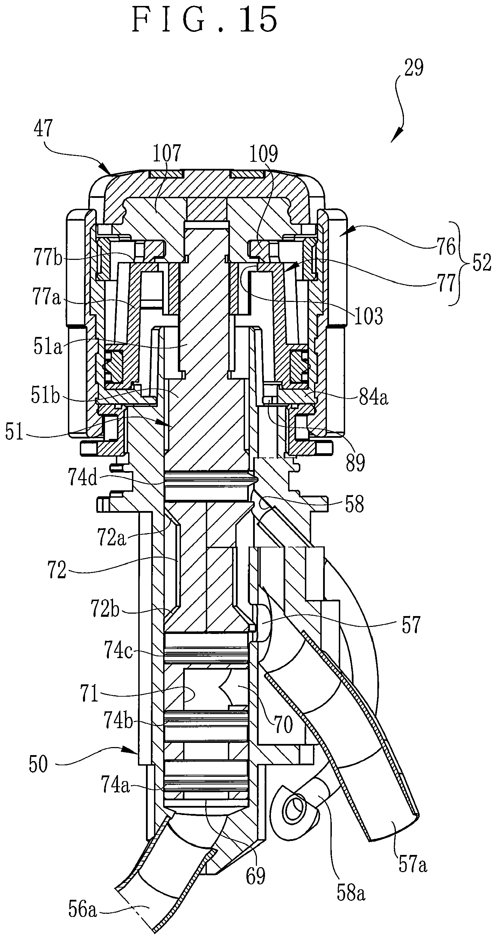

An annular channel 72 or annular flow opening is formed in a wall of the valve head 51b, disposed away from the valve opening 70 toward the end rod 51a, and extends in the longitudinal direction of the piston unit 51. The annular channel 72 includes an upper channel end 72a and a lower channel end 72b. The annular channel 72 has such a length that, when the piston unit 51 is depressed fully, the upper channel end 72a is opposed to the drain port 58, and that the lower channel end 72b is opposed to the flow opening 57. See Fig. 15. In Fig. 2, the annular channel 72 is partially cut away in the drawing for the purpose of clarifying the drain port 58.

A first packing 74a, second packing 74b, third packing 74c and fourth packing 74d are fitted on a wall of the valve head 51b. The first packing 74a is disposed at a lower end of the valve head 51b. The second packing 74b and the third packing 74c are disposed on the valve head 51b so that the valve opening 70 is positioned between those. The fourth packing 74d is disposed on the valve head 51b so that the annular channel 72 is positioned between the third packing 74c and the fourth packing 74d.

The piston unit 51 slides between an initial position and a down position. The button device 47, when the piston unit 51 is in the initial position, is not depressed but disposed the farthest from the end opening 55, and when the piston unit 51 is in the down position (full depression), is depressed fully, disposed the nearest to the end opening 55, and prevented from further moving down.

The piston unit 51, when in the initial position, is in a shut-off state to disconnect the flow opening 57 from the flow port 56 and the drain port 58 by use of the wall of the valve head 51b and the packing 74a-74d. The piston unit 51, when in the down position, is in a balloon deflation state of Fig. 15 for communicating the drain port 58 with the flow opening 57 by use of the annular channel 72. The piston unit 51, when slid to a halfway position between the initial position and down position by operating the button device 47, is in a suction state of Fig. 14 for communicating only the flow port 56 with the flow opening 57 by use of the flow channel 71.

The biasing unit 52 includes a cylinder cap device 76 of a cup shape or housing wall, an air-tight slide device 77 of a cup shape or guide cup by way of an intermediate casing, a first compression coil spring 78, and a second compression coil spring 79. The cylinder cap device 76 is secured to one end of the cylinder 50 and disposed around the end opening 55. The air-tight slide device 77 is disposed inside the cylinder cap device 76. Portions of the biasing unit 52 have a larger diameter than the end of the cylinder 50.

The cylinder cap device 76 is secured to an end of the cylinder 50 by the threaded ring 81. A receiving hole is formed in the threaded ring 81 for entry of the receiving threaded portion 60. A female thread (not shown) is provided on the inside of the receiving hole. The receiving threaded portion 60 or male thread is helically engaged with the female thread of the threaded ring 81 to connect the threaded ring 81 to the end of the cylinder 50. An annular flange 81a is formed on one end of the threaded ring 81.

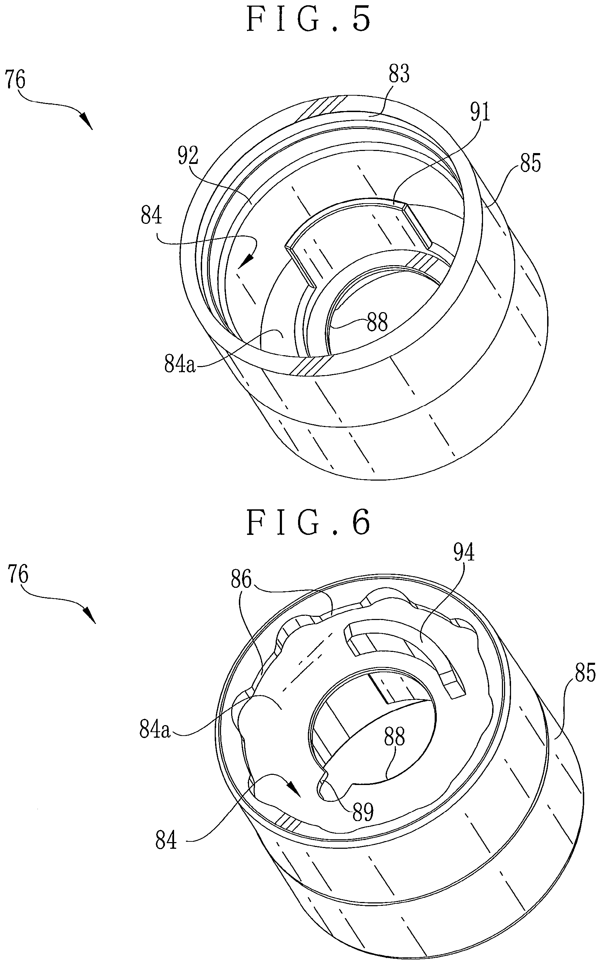

The cylinder cap device 76 includes a cup sleeve 84 and a cover sleeve 85. The cup sleeve 84 is formed from metal. The cover sleeve 85 is formed from resin, and covers the cup sleeve 84. A cup opening 83 is an upper open end of the cup sleeve 84 as illustrated in Fig. 5. A lower end of the cover sleeve 85 extends toward the cylinder 50 down under a bottom of the cup sleeve 84. Plural engaging teeth 86 are formed with an inner surface of the end of the cover sleeve 85 for engagement with the annular flange 81a. See Fig. 6. Thus, the cup sleeve 84 is connected to the cylinder 50 by the cover sleeve 85 and the threaded ring 81.

A bottom plate 84a of the cup sleeve 84 contacts an upper surface of the receiving threaded portion 60. A receiving hole 88 of Figs. 5 and 6 is formed through the bottom plate 84a for receiving entry of the cylinder 50. A cup vent hole 89 of Fig. 6 is formed in the bottom plate 84a, and opposed to the cylinder vent hole 65. Thus, air can flow between the inside of the cup sleeve 84 and the fluid channel 62 of the cylinder 50.

A sleeve wall 77a is included in the air-tight slide device 77 as illustrated in Figs. 2 and 3. In Fig. 5, a regulating ridge 91 is formed on a surface of the bottom plate 84a opposed to the air-tight slide device 77, and projects toward the sleeve wall 77a. An annular recess 92 for support is formed in an inner surface of the cup sleeve 84 and disposed under the cup opening 83.

In Fig. 6, a regulating ridge 94 is formed on a surface of the bottom plate 84a opposed to the cylinder 50, and projects toward the receiving threaded portion 60. The regulating ridge 94 is engaged with the regulating recess 64 of the receiving threaded portion 60. The regulating ridges 91 and 94 as illustrated in Fig. 2 are arranged collinearly with one another on a straight line extending in the longitudinal direction of the piston unit.

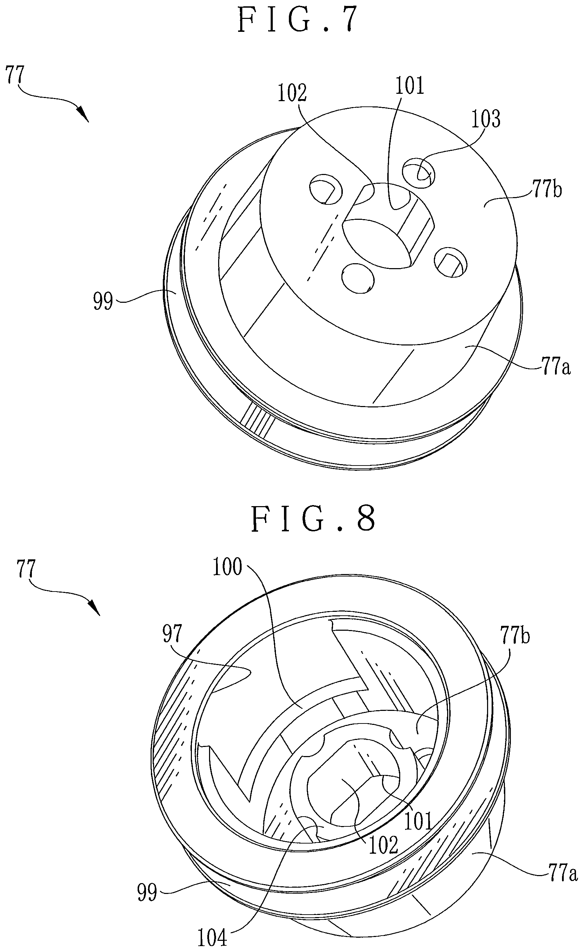

In Figs. 2 and 3, the air-tight slide device 77 is kept slidable inside the cup sleeve 84. An end ring 96 is secured to the annular recess 92, and maintains the air-tight slide device 77 inside the cup sleeve 84 without drop. The air-tight slide device 77 includes a cup plate portion 77b and the sleeve wall 77a. The sleeve wall 77a is cylindrical and extends longitudinally along the piston unit 51. The cup plate portion 77b is formed on an upper end of the sleeve wall 77a. A cup opening 97 is a lower open end of the sleeve wall 77a. See Fig. 8. Diameters of the sleeve wall 77a and the cup opening 97 are predetermined larger than an inner diameter of the end opening 55 and smaller than an inner diameter of the cup sleeve 84.

An annular seal packing 98 is fitted on the lower end of the sleeve wall 77a. A packing support 99 with two ring flanges of the sleeve wall 77a supports the annular seal packing 98. The annular seal packing 98 contacts an inner surface of the cup sleeve 84 and prevents leakage of air through a gap between an outer surface of the sleeve wall 77a and the inner surface of the cup sleeve 84. The ring flanges of the packing support 99 have an outer diameter larger than an inner diameter of the end ring 96.

A regulating recess 100 is formed in a second end portion of the sleeve wall 77a for engagement with the regulating ridge 91. See Fig. 8. A length of the regulating recess 100 is sufficiently larger than the regulating ridge 91 in the longitudinal direction of the piston unit 51 in order to keep the air-tight slide device 77 slidable without fail.

In Fig. 7, a receiving hole 101 is formed in the cup plate portion 77b and receives entry of the end rod 51a. A regulating flat surface 102 is formed flatly in the receiving hole 101 and is engaged with the regulating flat surface 67 of the end rod 51a. In short, the inner surface of the receiving hole 101 is non-circular in the presence of the regulating flat surface 102.

Plural cup vent holes 103 are formed in the cup plate portion 77b and disposed around the receiving hole 101. The cup vent holes 103 are open to the atmosphere. Thus, the discharge conduit 46 is caused to communicate with the atmosphere by the discharge channel coupling 57a, the fluid channel 62, the cup vent hole 89, the cup sleeve 84, the air-tight slide device 77, and the cup vent holes 103.

In Fig. 8, a guide sleeve 104 is formed on the cup plate portion 77b, and extends down toward the end opening 55. An upper end of the guide sleeve 104 has the receiving hole 101. The end rod 51a is inserted in the guide sleeve 104 in a slidable manner. As the guide sleeve 104 has a predetermined length in the longitudinal direction, the air-tight slide device 77 can be supported on the end rod 51a without an inclination. Offsetting of the piston unit 51 can be prevented when the piston unit 51 slides.

An inner diameter of the guide sleeve 104 is smaller than a diameter of the valve head 51b. A receiving surface 95 for retention is formed with a stepped shape on the valve head 51b. See Fig. 2. A lower end of the guide sleeve 104 contacts the receiving surface 95. This operates to keep the piston unit 51 positioned in the cylinder 50 without dropping by use of the air-tight slide device 77, the end ring 96, the cylinder cap device 76 and the threaded ring 81.

In Fig. 2, the air-tight slide device 77 is slidable between an upper position (protrusion position) and a lower position (storage position) . When the air-tight slide device 77 is in the upper position, an end of the packing support 99 contacts the end ring 96 to protrude the cup plate portion 77b from the cup opening 83. When the air-tight slide device 77 is in the lower position of Fig. 15, a second end of the sleeve wall 77a contacts the bottom plate 84a to contain the cup plate portion 77b in the cup opening 83. The air-tight slide device 77 is in the upper position while the piston unit 51 slides from the initial position to the halfway position.

When the piston unit 51 slides from the halfway position toward the down position upon depressing the button device 47, the air-tight slide device 77 is slid by the button device 47 from the upper position to the lower position. When the piston unit 51 comes to the down position, the air-tight slide device 77 is slid to the lower position, and prevents the button device 47 and the piston unit 51 from being pushed further.

The first compression coil spring 78 is disposed between the bottom plate 84a and the cup plate portion 77b in a compressed state along the piston unit 51 with a shorter length than its free state. The piston unit 51 is inserted through the first compression coil spring 78. The first compression coil spring 78 biases the cup plate portion 77b to protrude from the cup opening 83 so as to keep the air-tight slide device 77 in the upper position.

The second compression coil spring 79 is disposed between the packing support 99 and the button device 47 in a state compressed in the direction along the piston unit 51 with a reduced length. The piston unit 51 is inserted in the second compression coil spring 79. The second compression coil spring 79 biases the button device 47 in a direction to protrude from the receiving hole 101. Force of bias of the second compression coil spring 79 is structurally smaller than that of the first compression coil spring 78. When the button device 47 is depressed, at first the second compression coil spring 79 starts being deformed, then the first compression coil spring 78 starts being deformed. Consequently, the button device 47 can be moved and stopped in the halfway position owing to the difference in the force of bias between the compression coil springs 78 and 79.

The compression coil springs 78 and 79 are arranged inside the biasing unit 52 so that the air-tight slide device 77 is disposed between those. The first compression coil spring 78 inside the air-tight slide device 77 and the second compression coil spring 79 outside the air-tight slide device 77 constitute such a double structure that the biasing unit 52 can have a small height because the compression coil springs 78 and 79 can be accommodated compactly.

The piston unit 51 is maintained in the initial position by the bias of the compression coil springs 78 and 79. To slide the piston unit 51 from the initial position to the halfway position, the button device 47 must be pushed against the second compression coil spring 79. To slide the piston unit 51 from the halfway position to the down position (full depression), the button device 47 must be pushed against the compression coil springs 78 and 79. In short, the force of the bias applied to the button device 47 changes during the slide of the piston unit 51 from the initial position to the down position.

The button device 47 includes a cap top portion 106 of a disk shape and a button head or pressure ring 107 of metal. The cap top portion 106 is formed from resin. The button head 107 is secured to a lower surface of the cap top portion 106, and tightly contacts the cup plate portion 77b when the piston unit 51 is located between the halfway position and the down position. A ring projection 107a projects from the button head 107 toward the cup plate portion 77b. A screw hole 108 with a female thread (not shown) as retention mechanism is formed in the ring projection 107a. A male thread (not shown) of the end rod 51a is helically engaged with the screw hole 108, to fasten the button head 107 to the end rod 51a.

A pressure surface 110 or closure surface is formed with one end of the ring projection 107a, has an annular shape, and contacts the cup plate portion 77b. The pressure surface 110 covers and closes the cup vent holes 103 upon contacting the cup plate portion 77b. When the piston unit 51 is depressed fully, the pressure surface 110 contacts the cup plate portion 77b and the sleeve wall 77a contacts the bottom plate 84a. Each of those elements is formed from metal, so that errors in the piston longitudinal direction can be reduced in relation to the valve opening 70 or the annular channel 72 of the piston unit 51 in the down position.

An annular groove is formed in the ring projection 107a. A seal packing 109 of a ring shape is fitted in the annular groove, and formed from resilient material. An end of the seal packing 109 extends in a direction toward the cup plate portion 77b further than the pressure surface 110, and has a gradually decreasing thickness. When the pressure surface 110 contacts the cup plate portion 77b, the end of the seal packing 109 tightly contacts the cup plate portion 77b in a resiliently deformed state. Even when the cup vent holes 103 are not completely closed with the pressure surface 110, it is possible to disconnect the cup vent holes 103 from the atmosphere for shut-off of air. As a result, the discharge conduit 46 is shut off from the atmosphere.

Fig. 9 is a section taken on line IX-IX in Fig. 2. The regulating flat surface 67 of the end rod 51a is engaged with the regulating flat surface 102 of the receiving hole 101 of the cup plate portion 77b, so that the air-tight slide device 77 is rotationally regulated on the piston unit 51 about its axis.

Fig. 10 is a section taken on line X-X in Fig. 2. The regulating ridge 91 of the cup sleeve 84 is engaged with the regulating recess 100 of the sleeve wall 77a, so that the air-tight slide device 77 is rotationally regulated on the cylinder cap device 76 about its axis.

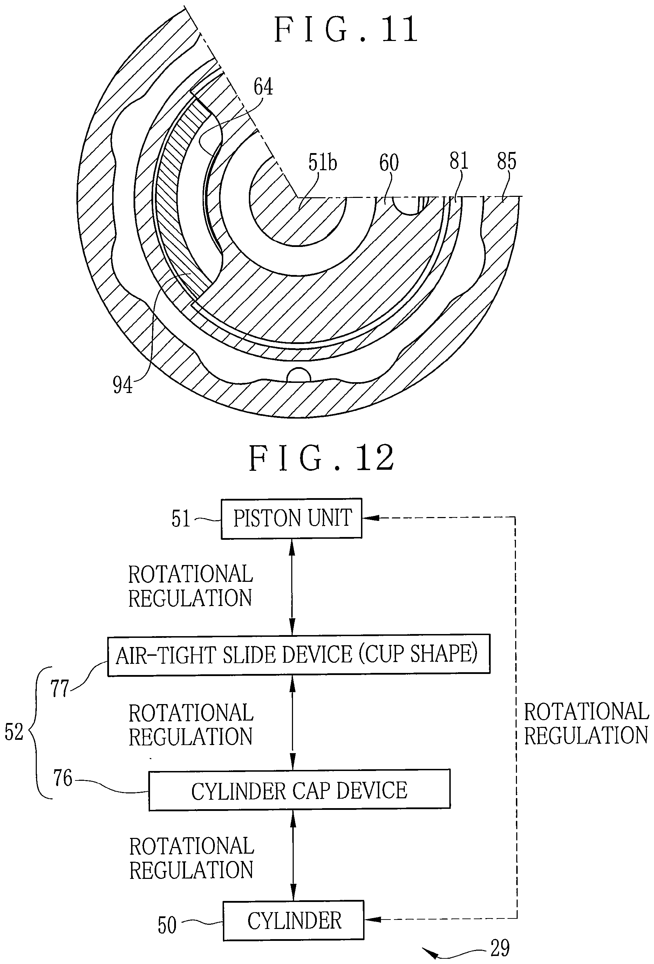

Fig. 11 is a section taken on line XI-XI in Fig. 2. The regulating ridge 94 of the bottom plate 84a is engaged with the regulating recess 64 of the receiving threaded portion 60, so that the cylinder cap device 76 is rotationally regulated on the cylinder 50 about its axis.

In Fig. 12, the rotational regulation is carried out between the piston unit 51 and the air-tight slide device 77, between the air-tight slide device 77 and the cylinder cap device 76, and between the cylinder cap device 76 and the cylinder 50. Thus, the piston unit 51 is rotationally regulated in the cylinder 50 by the biasing unit 52 indirectly. It is possible reliably to align the valve opening 70 with the flow opening 57 when the piston unit 51 is slid to the halfway position for the suction state, owing to the positions and shapes of the regulating flat surface 67, the regulating ridges 91 and 94, the regulating recesses 64 and 100 and the regulating flat surface 102.

The operation of the ultrasonic endoscope 10 is described now specifically in relation to the suction button assembly 29. In the endoscopic examination, a CCD image sensor and the ultrasonic transducer array 17 operate constantly. The air supply source 37 supplies air. The suction pump 45 carries out suction. When those devices are ready, the elongated tube 11 is entered in a body cavity of a patient, for example gastrointestinal tract, to start imaging. The balloon 21 is completely empty by removal of water, and remains compressed to contact the head assembly 11a tightly.

At first, a body part in the gastrointestinal tract is imaged endoscopically with the image sensor. The button device 43 of the air/water supply button assembly 33, if required for a type of the body part or for washing an imaging window (not shown) of the head assembly 11a, is operated to supply air and water through the air/water supply nozzle 18. When a doctor or operator wishes to change over the imaging, the endoscopic imaging is changed over to ultrasonic imaging, typically for more precise imaging upon discovery of a lesion in the gastrointestinal tract or the like.

For imaging with the ultrasonic endoscope, the button device 43 is depressed fully to supply water from the water supply source 22 through the water supply conduit 39, the balloon expansion channel 34 and the balloon channel 26 into the balloon 21, which is expanded. Various known methods for adjusting a flow rate of the water to the balloon 21 can be used in the embodiment. After the expansion, the balloon 21 is set in tight contact with an object of interest, for example, a lesion in a body part. An ultrasonic image of the object of interest is formed.

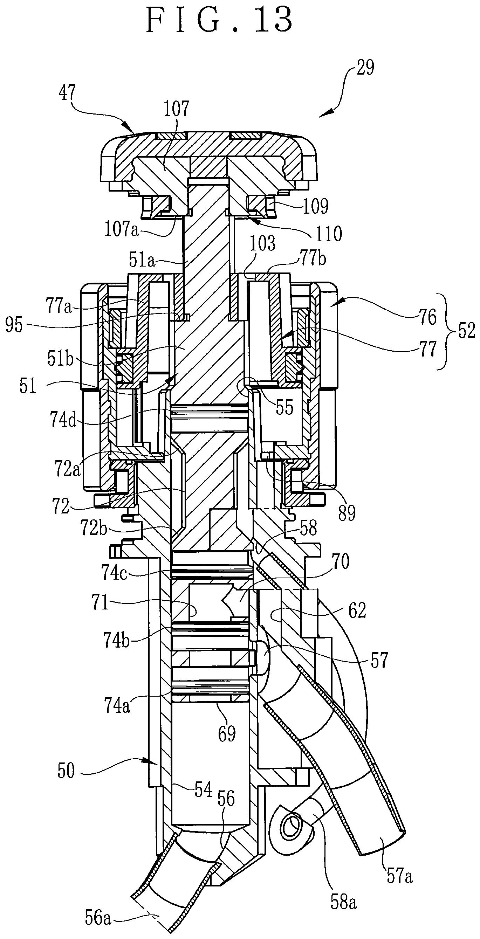

In Fig. 13, the button device 47 of the suction button assembly 29 is not pushed in a normal state without suction or drain of a balloon in the ultrasonic imaging or endoscopic imaging. The piston unit 51 is kept in the initial position for shut-off by the compression coil springs 78 and 79. As the flow channel 71 and the annular channel 72 have not been positioned for setting the flow opening 57 for communication of the flow port 56 with the drain port 58, the discharge conduit 46 is disconnected from the suction channel 28 and the balloon compression channel 35. There is no suction through the suction nozzle 19 or no drain of the balloon 21.

When the piston unit 51 is in the initial position, the cup vent holes 103 in the cup plate portion 77b are open. The flow opening 57 becomes open to the atmosphere through the fluid channel 62, the cup vent holes 89 and 103 and the like. It is possible to prevent overload to the suction pump 45 even when no suction is carried out through the suction nozzle 19 or when no removal of water is carried out from the balloon 21.

If suction of blood or body fluid is required in the imaging, the button device 47 is depressed halfway to slide in the piston unit 51 through the end opening 55. Before the piston unit 51 reaches the halfway position, force of bias of the second compression coil spring 79 is applied to the button device 47. After the piston unit 51 slides down past the halfway position, force of bias of both the compression coil springs 78 and 79 is applied to the button device 47. In short, the force of bias applied to the button device 47 increases, so that the piston unit 51 can be stopped suitably in the halfway position.

In Fig. 14, the piston unit 51 is changed over from the inactive state to the suction state upon stop in the halfway position. In the suction state, the valve opening 70 of the flow channel 71 becomes aligned with the flow opening 57. The lower channel end 72b of the annular channel 72 has not reached the position of the flow opening 57. Also, the third packing 74c comes to a location between the flow opening 57 and the drain port 58. As a result, only the flow port 56 comes to communicate with the flow opening 57.

When the flow port 56 comes to communicate with the flow opening 57, the discharge conduit 46 is set in series with the suction channel 28 and the instrument channel 24 by the flow channel 71 and other channels. When the piston unit 51 is in the halfway position, the pressure surface 110 of the ring projection 107a and the seal packing 109 contact the cup plate portion 77b tightly to close the cup vent holes 103. The discharge conduit 46 is shut off from the atmosphere, to raise suction force of the negative pressure in the channels including the discharge conduit 46 and the suction nozzle 19. Thus, fluid is drawn through the suction nozzle 19 by the suction. The fluid is passed through the instrument channel 24, the suction channel 28, the cylinder passage 54, the flow channel 71 and the discharge conduit 46 and discharged from the ultrasonic endoscope 10.

Consequently, the valve opening 70 of the piston unit 51 is always aligned with the flow opening 57 because the biasing unit 52 prevents the piston unit 51 from rotating relative to the cylinder 50 upon changeover of the piston unit 51 to the suction state. A flow channel width determined between the valve opening 70 and the flow opening 57 is maximized for flow of fluid, so as to increase the performance of suction of the suction button assembly 29 very effectively. Also, it is unnecessary to dispose a rotationally regulating element to each one of a wall of the piston unit and an inner wall of the cylinder. The degree of freedom of defining positions of the flow channel 71, the annular channel 72 and other channels can be high in the piston unit 51. A size of the piston unit 51 or the suction button assembly 29 can be reduced.

To discontinue the suction, the button device 47 is left without depression. The second compression coil spring 79 returns the piston unit 51 to the initial position of Fig. 13.

When the ultrasonic imaging is terminated, the button device 47 is depressed fully to slide the piston unit 51 down into the end opening 55. Before the piston unit 51 comes to reach the halfway position, force of bias of the second compression coil spring 79 is applied to the button device 47. When the piston unit 51 moves past the halfway position, force of bias of the compression coil springs 78 and 79 is applied to the button device 47. Also, when the piston unit 51 moves down past the halfway position, pressure of the button device 47 moves the air-tight slide device 77 from the upper position to the lower position. When the button device 47 continues being pushed against the compression coil springs 78 and 79, the air-tight slide device 77 reaches the lower position, and prevents the button device 47 from moving down further. Thus, the piston unit 51 is stopped in the down position (full depression).

In Fig. 15, the piston unit 51 is in the down position for changeover to the balloon deflation state. In this state, the valve opening 70 of the flow channel 71 is offset from the flow opening 57 in the longitudinal direction of the piston unit 51. The annular channel 72 is shifted to oppose the upper channel end 72a to the drain port 58 and the lower channel end 72b to the flow opening 57. Also, the third packing 74c shifts to a point between the valve opening 70 and the flow opening 57. Only the drain port 58 is set in series with the flow opening 57.

When the drain port 58 comes to communicate with the flow opening 57, the discharge conduit 46 and the balloon compression channel 35 (and the balloon channel 26) are caused to communicate with one another by the annular channel 72. As the cup vent holes 103 are closed in a manner similar to the suction described above, the discharge conduit 46 is shut off from the atmosphere. Force of suction with the negative pressure rises within the channels including the discharge conduit 46 and the balloon channel 26. Thus, water is removed from the balloon 21 which is compressed. The removed water is drawn through the balloon channel 26, the balloon compression channel 35, the annular channel 72 and the discharge conduit 46 and drained from the ultrasonic endoscope 10.

Discharge of a predetermined amount of water from the balloon 21 is detected according to a known method of detection. In response, the button device 47 is released from being pushed. Thus, the piston unit 51 is returned to the initial position of Fig. 13 by the bias of the compression coil springs 78 and 79.

Similarly, the air/water supply button assembly 33 and the suction button assembly 29 are actuated suitably until the end of the examination with the ultrasonic endoscope 10. Supply of air and water, water supply to the balloon, suction, water drain from the balloon are carried out.

In the above embodiment, the annular seal packing 98 is provided on the air-tight slide device 77. However, the annular seal packing 98 can be omitted. A preferred air-tight slide device can have an outer surface in tight contact with an inner surface of the cylinder cap device 76 in a slidable form in an air-tight manner without a vent passage.

In the embodiment, the piston unit 51 in the suction button assembly 29 is prevented from rotating relative to the cylinder 50. However, it is possible according to the invention to prevent the piston unit 51 in the air/water supply button assembly 33 from rotating relative to the cylinder 50.

In the above embodiment, the piston unit 51 is prevented from rotating relative to the cylinder 50 by use of the regulating flat surface 67, the regulating ridges 91 and 94, the regulating recesses 64 and 100 and the regulating flat surface 102. However, various other non-circular forms for preventing rotation can be used as regulating structures according to the invention.

In the suction button assembly 29 of the embodiment, three channels including the suction channel 28, the balloon compression channel 35 and the discharge conduit 46 are formed for changeover. Furthermore, the number of the channels formed in the suction button assembly 29 of the invention can be four or more.

In the above embodiment, the endoscope is the ultrasonic endoscope 10. However, an endoscope of the invention with the suction button assembly 29 can be any of various types, for example, colonoscope for entry in a large intestine.

## Claims

1. A switching valve assembly for an endoscope (10), including a cylinder (50) having a cylinder passage (54) and a plurality of flow openings (56, 57) formed inside said cylinder passage, plural flow lines (28, 46) extending from respectively said flow openings, said switching valve assembly comprising:
a piston unit (51) contained in said cylinder passage in a slidable manner, said piston unit including a valve head (51b), a flow channel (71, 72) formed in said valve head, an end rod (51a), formed to extend from said valve head in an upward direction, for protruding from said cylinder passage, and a button device (43, 47) formed on a tip of said end rod;
wherein said piston unit slides to an initial position, a down position lower than said initial position and where said button device 47 is positioned nearest to said cylinder passage, and a halfway position defined between said initial position and said down position, for changing a combination of said flow openings with said flow channel to change over communication and interruption between said flow lines;
a biasing means (52) of a two-stage type, secured to said cylinder on an upper side of said cylinder passage, for biasing said piston unit in said upward direction from said down position, said biasing means having smaller force of bias above said halfway position than under said halfway position, for stopping said piston unit in said halfway position when said button device is pushed from said initial position and slid in a downward direction;
regulating means adapted to prevent relative rotation between the piston unit and the cylinder, the regulating means being **characterised by**
a first regulating means (67, 102) adapted to prevent said piston unit from rotating relative to said biasing means; and
a second regulating means (64, 94) adapted to preventing said biasing means from rotating relative to said cylinder, so as to ensure alignment of a channel opening of said flow channel with one of said flow openings upon sliding said piston unit.

2. A switching valve assembly as defined in claim 1, wherein said biasing means includes:
a cylinder cap device (76), secured to said cylinder under said button device, for partially covering said piston unit;
a slide device (77), of a cup shape, contained in said cylinder cap device, having a receiving hole (101), for receiving entry of said piston unit, for guiding said piston unit in a longitudinal direction thereof, and for sliding in said downward direction in said cylinder cap device when said button device is pushed in said downward direction;
air-tight packing (98), fitted on said slide device, for contacting an inner surface of said cylinder cap device in an air-tight manner;
a first biasing mechanism (78), disposed between said slide device and said cylinder cap device, for biasing said slide device in said upward direction from said cylinder;
a second biasing mechanism (79), disposed between said button device and said slide device, for biasing said button device in said upward direction with smaller force of bias than said first biasing mechanism.

3. A switching valve assembly as defined in claim 2, wherein said first and second biasing mechanisms are compression coil springs.

4. A switching valve assembly as defined in claim 2, further comprising a third regulating means (91,100), for preventing said slide device from rotating relative to said cylinder cap device;
wherein said first regulating means prevents said piston unit from rotating relative to said slide device, and said second regulating means prevents said cylinder cap device from rotating relative to said cylinder.

5. A switching valve assembly as defined in claim 4, wherein said first regulating means includes:
a first regulating flat surface formed by chamfering a peripheral surface of said end rod;
a second regulating flat surface, formed inside said receiving hole, for tightly contacting said first regulating flat surface for engagement.

6. A switching valve assembly as defined in claim 4, wherein said third regulating means includes:
a first regulating projection (91), formed to project from a bottom plate of said cylinder cap device in said upward direction;
a first regulating recess, formed in an inner wall of said slide device, and engaged with said first regulating projection.

7. A switching valve assembly as defined in claim 6, wherein said second regulating means includes:
a second regulating projection, formed to project from said bottom plate of said cylinder cap device in said downward direction;
a second regulating recess, formed in said cylinder, and engaged with said second regulating projection.

8. A switching valve assembly as defined in claim 7, wherein said first regulating projection is disposed collinearly with said second regulating projection in said longitudinal direction of said piston unit.

9. A switching valve assembly as defined in claim 2, further comprising an end ring (96), secured inside said cylinder cap device, for keeping said slide device contained in said cylinder cap device.

10. A switching valve assembly as defined in claim 2, further comprising a guide sleeve (104), disposed on an upper wall of said slide device, having said receiving hole, for extending long in said longitudinal direction of said piston unit.

11. A switching valve assembly as defined in claim 2, wherein said valve head (51b) has a larger diameter than a diameter of said receiving hole (101).

12. A switching valve assembly as defined in claim 2, wherein said button device includes:
a button head (107) secured to said tip of said end rod (51a) ; a cap top portion (100) for covering an upper surface of said button head.

## Patentansprüche

1. Schaltventilanordnung für ein Endoskop (10), enthaltend einen Zylinder (50) mit einem Zylinderkanal (54) und mehreren Strömungsöffnungen (56, 57), die innerhalb des Zylinderkanals ausgebildet sind, und mehrere Strömungsleitungen (28, 46), die sich von den jeweiligen Strömungsöffnungen aus erstrecken, umfassend:
eine Kolbeneinheit (51), die in dem Zylinderkanal gleitfähig enthalten ist, wobei die Kolbeneinheit einen Ventilkopf (51b), einen in dem Ventilkopf ausgeformten Strömungskanal (71, 72), eine sich von dem Ventilkopf in Aufwärtsrichtung erstreckende Endstange (51a) zum Herausragen aus dem Zylinderkanal, und eine am Ende der Endstange ausgeformte Knopfeinrichtung (43, 47) aufweist;
wobei die Kolbeneinheit in eine Anfangsposition, eine Untenposition tiefer als die Anfangsposition und mit der Knopfeinrichtung (47) dem Zylinderkanal am nächsten gelegen, und einer Halbposition zwischen der Anfangsposition und der Untenposition verschieblich ist, um eine Kombination der Strömungsöffnungen mit dem Strömungskanal und damit eine Verbindung und eine Unterbrechung zwischen den Strömungsleitungen zu ändern;
eine zweistufige Vorspanneinrichtung (52), die an einer Oberseite des Zylinderkanals festgelegt ist, um die Kolbeneinheit aus der Untenposition in die Aufwärtsrichtung vorzuspannen, wobei die Vorspanneinrichtung eine geringere Vorspannkraft oberhalb der Halbposition als unterhalb der Halbposition besitzt, um die Kolbeneinheit in der Halbposition anzuhalten, wenn die Knopfeinrichtung aus der Anfangsposition herausgedrückt wird und in Abwärtsrichtung gleitet;
eine Reguliereinrichtung, ausgebildet zum Verhindern einer Relativdrehung zwischen der Kolbeneinheit und dem Zylinder, wobei die Reguliereinrichtung **gekennzeichnet ist durch**
eine erste Reguliereinrichtung (67, 102), ausgebildet zum Verhindern, dass die Kolbeneinheit sich relativ zu der Vorspanneinrichtung dreht; und
eine zweite Reguliereinrichtung (64, 94), ausgebildet zum Verhindern, dass die Vorspanneinrichtung sich relativ zu dem Zylinder dreht, um eine Fluchtung einer Kanalöffnung des Strömungskanals mit einer der Strömungsöffnungen beim Gleiten der Kolbeneinheit zu gewährleisten.

2. Schaltventilanordnung nach Anspruch 1, wobei die Vorspanneinrichtung enthält:
eine Zylinderkappeneinrichtung (76), die an dem Zylinder unterhalb der Knopfeinrichtung festgelegt ist, um die Kolbeneinheit teilweise abzudecken;
eine Gleiteinrichtung (77) in Becherform, die in der Zylinder-Kappeneinrichtung enthalten ist, ein Aufnahmeloch (101) zum Aufnehmen des Eintritts der Kolbeneinheit besitzt, um die Kolbeneinheit in deren Längsrichtung zu führen, und um in Abwärtsrichtung innerhalb der Zylinderkappeneinrichtung zu gleiten, wenn die Knopfeinrichtung in der Abwärtsrichtung gedrückt wird;
eine luftdichte Packung (98), die an der Gleiteinrichtung angepasst ist, um in luftdichter Weise mit einer Innenfläche der Zylinderkappeneinrichtung in Berührung zu treten;
einen ersten Vorspannmechanismus (78), der sich zwischen der Gleiteinrichtung und der Zylinderkappeneinrichtung befindet, um die Gleiteinrichtung in Aufwärtsrichtung von dem Zylinder weg vorzuspannen;
einen zweiten Vorspannmechanismus (79), der sich zwischen der Knopfeinrichtung und der Gleiteinrichtung befindet, um die Knopfeinrichtung in der Aufwärtsrichtung mit einer geringeren Vorspannkraft als der erste Vorspannmechanismus vorzuspannen.

3. Schaltventilanordnung nach Anspruch 2, bei der der erste und der zweite Vorspannmechanismus Druck-Schraubenfedern sind.

4. Schaltventilanordnung nach Anspruch 2, weiterhin umfassend eine dritte Reguliereinrichtung (91, 100) zum Verhindern, dass die Gleiteinrichtung sich relativ zu der Zylinderkappeneinrichtung dreht;
wobei die erste Reguliereinrichtung verhindert, dass die Kolbeneinrichtung sich relativ zu der Gleiteinrichtung dreht, und die zweite Reguliereinrichtung verhindert, dass die Kappeneinrichtung sich relativ zu dem Zylinder dreht.

5. Schaltventilanordnung nach Anspruch 4, bei der die erste Reguliereinrichtung enthält:
eine erste Regulier-Flachseite, gebildet durch Abfasen einer Umfangsfläche der Endstange;
eine zweite Regulier-Flachseite, ausgebildet im Inneren des Aufnahmelochs, um zusammenwirkend die erste Regulierflachseite festzukontaktieren.

6. Schaltventilanordnung nach Anspruch 4, bei der die dritte Reguliereinrichtung enthält:
einen ersten Reguliervorsprung (91), ausgebildet in der Weise, dass er von einer Bodenplatte der Zylinderkappeneinrichtung in der Aufwährtsrichtung vorsteht;
eine erste Reguliervertiefung, ausgebildet in einer Innenwand der Gleiteinrichtung und zusammenwirkend mit dem ersten Reguliervorsprung.

7. Schaltventilanordnung nach Anspruch 6, bei der die erste Reguliereinrichtung enthält:
einen zweiten Reguliervorsprung, ausgebildet in der Weise, dass er von der Bodenplatte der Zylinderkappeneinrichtung in Abwärtsrichtung absteht;
eine zweite Regulierausnehmung, die in dem Zylinder ausgebildet ist und in Eingriff steht mit dem zweiten Reguliervorsprung.

8. Schaltventilanordnung nach Anspruch 7, bei der der erste Reguliervorsprung sich auf einer Linie mit dem zweiten Reguliervorsprung in Längsrichtung der Kolbeneinheit befindet.

9. Schaltventilanordnung nach Anspruch 2, weiterhin umfassend einen Endring (96), der im Inneren der Zylinderkappeneinrichtung festgelegt ist, um die Gleiteinrichtung in der Zylinderkappeneinrichtung zu halten.

10. Schaltventilanordnung nach Anspruch 2, weiterhin umfassend eine Führungshülse (104), die sich an einer oberen Wand der Gleiteinrichtung befindet und das Aufnahmeloch enthält, um sich entlang der Längsrichtung der Kolbeneinheit zu erstrecken.

11. Schaltventilanordnung nach Anspruch 2, bei der der Ventilkopf (51b) einen größeren Durchmesser hat als das Aufnahmeloch (101).

12. Schaltventilanordnung nach Anspruch 2, bei der die Knopfeinrichtung enthält:
einen Kopf (107), der am Ende der Endstange (51a) angebracht ist;
ein Kappen-Oberteil (100) zum Bedecken der Oberseite des Kopfs.

## Revendications

1. Ensemble formant soupape de commutation destiné à un endoscope (10),
incluant un cylindre (50) présentant un passage de cylindre (54) et une pluralité d'ouvertures d'écoulement (56, 57) formées dans ledit passage de cylindre, et plusieurs lignes d'écoulement (28, 46) s'étendant respectivement à partir desdites ouvertures d'écoulement, ledit ensemble formant soupape de commutation comprenant :
une unité de piston (51) contenue dans ledit passage de cylindre d'une manière permettant le coulissement, ladite unité de piston incluant une tête de soupape (51b), un canal d'écoulement (71, 72) formé dans ladite tête de soupape, une tige d'extrémité (51a), formée de manière à s'étendre à partir de ladite tête de soupape dans une direction ascendante, pour faire saillie à partir dudit passage de cylindre, et un dispositif de bouton (43, 47) formé sur une pointe de ladite tige d'extrémité ;
dans lequel ladite unité de piston coulisse vers une position initiale, une position basse inférieure à ladite position initiale et où ledit dispositif de bouton (47) est positionné au plus près dudit passage de cylindre, et une position à mi-chemin définie entre ladite position initiale et ladite position basse, pour modifier une combinaison desdites ouvertures d'écoulement avec ledit canal d'écoulement pour une permutation entre communication et interruption entre lesdites lignes d'écoulement ;
un moyen de sollicitation (52) de type à deux étapes, fixé sur ledit cylindre sur un côté supérieur dudit passage de cylindre, destiné à solliciter ladite unité de piston dans ladite direction ascendante à partir de ladite position basse, ledit moyen de sollicitation présentant une force de sollicitation plus petite au-dessus de ladite position à mi-chemin que sous ladite position à mi-chemin, et à arrêter ladite unité de piston dans ladite position à mi-chemin lorsque ledit dispositif de bouton est poussé à partir de ladite position initiale et coulissé dans une direction descendante ;
un moyen de régulation, apte à empêcher la rotation relative entre l'unité de piston et le cylindre, le moyen de régulation étant **caractérisé par**
un premier moyen de régulation (67, 102) apte à empêcher la rotation relative de ladite unité de piston par rapport audit moyen de sollicitation, et
un deuxième moyen de régulation (64, 94) apte à empêcher la rotation relative dudit moyen de sollicitation par rapport audit cylindre, de manière à assurer un alignement d'une ouverture de canal dudit canal d'écoulement avec une desdites ouvertures d'écoulement lors du coulissement de ladite unité de piston.

2. Ensemble formant soupape de commutation selon la revendication 1, dans lequel ledit moyen de sollicitation inclut :
un dispositif de couvercle de cylindre (76), fixé audit cylindre sous ledit dispositif de bouton, pour couvrir partiellement ladite unité de piston ;
un dispositif de glissière (77) en forme de coupelle, contenu dans ledit dispositif de couvercle de cylindre, présentant un trou récepteur (101), destiné à recevoir l'entrée de ladite unité de piston, à guider ladite unité de piston dans une direction longitudinale de celle-ci, et à coulisser dans une direction descendante dans ledit dispositif de couvercle de cylindre lorsque ledit dispositif de bouton est poussé dans ladite direction descendante ;
une garniture étanche à l'air (98), montée sur ledit dispositif de glissière, pour venir en contact avec une surface intérieure dudit dispositif de couvercle de cylindre d'une manière étanche à l'air.
un premier mécanisme de sollicitation (78), disposé entre ledit dispositif de glissière et ledit dispositif de couvercle de cylindre, destiné à solliciter ledit dispositif de glissière dans ladite direction ascendante à partir dudit cylindre ;
un second mécanisme de sollicitation (79), disposé entre ledit dispositif de bouton et ledit dispositif de glissière, destiné à solliciter ledit dispositif de bouton dans ladite direction ascendante avec une force de sollicitation plus petite que pour ledit premier mécanisme de sollicitation.

3. Ensemble formant soupape de commutation selon la revendication 2, dans lequel lesdits premier et second mécanismes de sollicitation sont des ressorts à enroulement de compression.

4. Ensemble formant soupape de commutation selon la revendication 2, comprenant en outre un troisième moyen de régulation (91, 100) destiné à empêcher la rotation relative dudit dispositif de glissière par rapport audit dispositif de couvercle de cylindre ;
dans lequel ledit premier moyen de régulation empêche la rotation relative de ladite unité de piston par rapport audit dispositif de glissière, et ledit deuxième moyen de régulation empêche la rotation relative dudit dispositif de couvercle de cylindre par rapport audit cylindre.

5. Ensemble formant soupape de commutation selon la revendication 4, dans lequel ledit premier moyen de régulation inclut :
une première surface plate de régulation formée en chanfreinant une surface périphérique de ladite tige d'extrémité, et
une seconde surface plate de régulation, formée dans ledit trou récepteur, pour un contact étroit de ladite première surface plate de régulation en vue d'une prise.

6. Ensemble formant soupape de commutation selon la revendication 4, dans lequel ledit troisième moyen de régulation inclut :
une première saillie de régulation (91), formée pour faire saillie à partir d'une plaque inférieure dudit dispositif de couvercle de cylindre dans ladite direction ascendante, et
un premier retrait de régulation, formé dans une paroi intérieure dudit dispositif de glissière, et en prise avec ladite première saillie de régulation.

7. Ensemble formant soupape de commutation selon la revendication 6, dans lequel ledit deuxième moyen de régulation inclut :
une seconde saillie de régulation, formée pour faire saillie à partir de ladite plaque inférieure dudit dispositif de couvercle de cylindre dans ladite direction descendante, et
un second retrait de régulation, formé dans ledit cylindre, et en prise avec ladite seconde saillie de régulation.

8. Ensemble formant soupape de commutation selon la revendication 7, dans lequel ladite première saillie de régulation est disposée de manière colinéaire avec ladite seconde saillie de régulation dans ladite direction longitudinale de ladite unité de piston.

9. Ensemble formant soupape de commutation selon la revendication 2, comprenant en outre un anneau d'extrémité (96), fixé dans ledit dispositif de couvercle de cylindre, pour maintenir ledit dispositif de glissière contenu dans ledit dispositif de couvercle de cylindre.

10. Ensemble formant soupape de commutation selon la revendication 2, comprenant en outre un manchon de guidage (104), disposé sur une paroi supérieure dudit dispositif de glissière, présentant ledit trou récepteur, destiné à s'étendre en longueur dans ladite direction longitudinale de ladite unité de piston.

11. Ensemble formant soupape de commutation selon la revendication 2, dans lequel ladite tête de soupape (51b) présente un diamètre plus grand qu'un diamètre dudit trou récepteur (101).

12. Ensemble formant soupape de commutation selon la revendication 2, dans lequel ledit dispositif de bouton inclut :
une tête de bouton (107) fixée sur ladite pointe de ladite tige d'extrémité (51a), et
une portion de dessus de couvercle (100) pour couvrir une surface supérieure de ladite tête de bouton.
